# FASCICULE DE BREVET EUROPEEN

(11) **EP 2 456 776 B1**
(45) Date de publication et mention de la délivrance du brevet: **27.01.2016**
(21) Numéro de dépôt: 10752081.9
(22) Date de dépôt: 22.07.2010
(51) Int. Cl.: C07F 17/00, A61P 35/00

(54) **COMPLEXES ORGANOMETALLIQUES ACTIFS DERIVES DE SÉLÉNOQUINONE, LEURS PROCEDES DE SYNTHESE ET UTILISATIONS**
SELENCHINON-ABGELEITETE AKTIVE ORGANOMETALLISCHE KOMPLEXE, SYNTHESEVERFAHREN DAFÜR UND VERWENDUNGEN DAVON
SELENOQUINONE-DERIVED ACTIVE ORGANOMETALLIC COMPLEXES, METHODS FOR SYNTHESIZING SAME, AND USES THEREOF

(30) Priorité: 23.07.2009 FR 0903641
(43) Date de publication de la demande: 30.05.2012
(73) Titulaire: Centre National de la Recherche Scientifique (CNRS), 75794 Paris Cedex 16 (FR)
(72) Inventeur: AMOURI, Hani, Haniel, F-75013 Paris (FR)
(74) Mandataire: Novagraaf Technologies
(86) Numéro de dépôt international: PCT/FR2010/051555
(87) Numéro de publication internationale: WO 2011/010072

(56) Documents cités:
- ES-A1- 2 323 523
- JP-A- 7 089 855
- US-A- 5 760 266
- MOUSSA J., RAGER M. N., CHAMOREAU L. M., RICARD L., AMOURI H.: "Unprecedented pi-Bonded Rhodio- and Iridio-o-Benzoquinones as Organometallic Linkers for the Design of Chiral Octahedral Bimetallic Assemblies" ORGANOMETALLICS, vol. 28, 2 janvier 2009 (2009-01-02), pages 397-404, XP002562782
- MILLER E. J., LANDON S. J., BRILL T. B.: "n5-C5(CH3)5 vs. n5-C5H5. A Comparison of Electronic Influences for Metallocenes with fac-a3b2c, fac-a3b3, and cis-a3b2 Ligand Geometry Based on 59Co NQR Spectroscopy" ORGANOMETALLICS, vol. 4, 1985, pages 533-538, XP002562783
- NAGAO S., SEINO H., OKADA T., MIZOBE Y., HIDAI M.: "Syntheses of diiridium complexes with two bridging tetrachalcogenide ligands [{Ir(nu5-C5Me5)}2(mu-E4)2] (E= Se or S) and their reactions with alkynes forming mono- or di-nuclear dichalcogenolene complexes" DALTON, 2000, pages 3546-3553, XP009127896

## Description

### Domaine technique

La présente invention est relative à la synthèse de complexes organométalliques biologiquement actifs à partir de sélénoquinone.

Ces complexes trouvent des applications dans le domaine de la prévention ou du traitement de maladies impliquant une prolifération cellulaire anormale, notamment du cancer.

Dans la description ci-dessous, les références entre crochets (**[]**) renvoient à la liste des références présentée à la fin du texte.

### Etat de la technique

Les quinones représentent une classe de composés jouant un rôle important en chimie et en biologie **[1]**. Par exemple, la vitamine K possède une fonction quinone dans sa structure (naphtoquinone) **[2]**, et possède des propriétés anti-hémorragiques, la Lawsone est une naphtoquinone produite à l'état naturel par un plante originaire d'Arabie (Lawsonia Inermis) plus connue sous le nom de Henné et utilisée notamment pour teindre la laine et la soie, ou encore les cheveux, l'alizarine est un colorant naturel de couleur rouge synthétisé par W.H. Perkin en 1868.

L'activité biologique des quinones est souvent associée à leurs niveaux de transfert électronique et à leur potentiel d'oxydo-réduction **[3]**. Les couples redox quinone/hydroquinone ont été largement utilisés dans les études électrochimiques du fait de leur disponibilité rapide et qu'ils présentent un bon comportement électrochimique **[4]**.

L'hydroquinone en solution aqueuse a de nombreuses utilisations principalement en raison de son action comme agent réducteur. C'est l'un des composants majeurs dans le développement photographique où, en présence de Metol (ou 4-(méthylamino)phénol), elle réduit les sels d'argent exposés, invisibles, en argent métallique. En médecine humaine, l'hydroquinone a été utilisée en application sur la peau pour en réduire la couleur sans risque de dermatoses, via la diminution de la synthèse de mélanine par inhibition de la formation de l'enzyme tyrosinase. Toutefois cet usage est interdit dans l'Union européenne depuis février 2001, par crainte d'effets cancérigènes et de « complications graves ».

Actuellement, il existe très peu d'exemples de complexes organométalliques où l'hydroquinone ou la quinone agissent comme des ligands «π-liés» (π-bonded) **[5,6]**. Ainsi, l'analogue du chrome Cr(CO)₃(η⁶-hydroquinone) a été signalé comme thermiquement instable et sensible à l'air, et de ce fait ne pouvait être isolé **[7]**. Par conséquent, la nature du métal et des ligands auxiliaires semble jouer un rôle important dans la stabilisation de ces complexes quinone/hydroquinone.

En 1998, les Inventeurs ont réussi la synthèse des premiers complexes métalliques quinone/hydroquinone stables : le complexe iridium hydroquinone stable [(C₅Me₅)Ir(η⁶-hydroquinone)]²⁺ et le complexe iridium quinone [(C₅Me₅)Ir(η⁴-quinone)] **[8]**. Puis en 2004, ils ont réussi la synthèse de complexes rhodium hydroquinone/quinone [(C₅Me₅)Rh(η⁶-hydroquinone)]²⁺ et [(C₅Me₅)Rh(η⁴-quinone)] **[9]**.

Contrairement aux benzoquinones (*ortho-* et *para*-)*,* leurs analogues soufrés, les dithiobenzoquinones (*ortho-* et *para*-) sont des intermédiaires réactifs très instables et demeurent par conséquent très peu étudiés comme en atteste le très faible nombre de publications à ce sujet. La raison essentielle de l'instabilité des dithiobenzoquinones trouve ses racines dans la grande labilité générale de la double liaison C=S par rapport à la double liaison C=O **[10]**. Cependant, en 2006 et 2007, les Inventeurs ont réussi par un procédé de synthèse novateur, la synthèse de complexes organométalliques dithioquinone stables sous forme d'isomères *ortho-* et *para-* **[12]**.

Cependant, contrairement aux propriétés biologiques des quinones libres, celles des complexes organométalliques dérivés de quinone/hydroquinone, et leurs dérivés souffrés n'ont pas été identifiées.

Parmi les complexes organométalliques dont les propriétés biologiques ont été mises en évidence, on peut citer le cis-platine ou cis-diaminedichloroplatine(II) (CDDP), qui est un complexe à base de platine utilisée dans le traitement de différents cancers tels les sarcomes, carcinomes (cancer du poumon à petites cellules, cancer de l'ovaire...), lymphomes. Il appartient à la classe des composés alkylant l'ADN avec le carboplatine et l'oxaliplatine. Le cis-platine est un complexe organométallique qui se fixe sélectivement sur les bases puriques de l'ADN (A ou G) et induit une variation de la conformation locale du double brin d'ADN. Cette déformation inhibe la réplication et la transcription de l'ADN en ARN, et induit par ce biais la mort cellulaire. Différents mécanismes protéiques de réparations de vis-à-vis de la formation d'adduits de cis-platine-ADN existent et reconnaissent certains des adduits formés. La recherche contre le cancer s'appuie sur la cytotoxicité du cis-platine, tout en cherchant de nouveaux moyens de cibler la toxicité sur les cellules cancéreuses (dépourvues de certains mécanismes de contrôle de l'ADN) **[14]**.

Le cancer est l'une des causes de mortalité les plus importantes et par conséquent l'un des problèmes de santé publique les plus graves dans le monde d'aujourd'hui. De nombreux médicaments ont été et sont développés. Cependant, ces médicaments ne permettent pas de traiter tous les cas avec succès. D'autre part, les drogues utilisées dans le cadre des chimiothérapies peuvent présenter des effets secondaires indésirables, une efficacité et/ou une spécificité d'action vis-à-vis des cellules cancéreuses insuffisante.

En particulier, le cis-platine présente des effets secondaires toxiques non négligeables, et peut entraîner des allergies, des troubles gastro-intestinales (nausées, vomissements, ulcerations digestives), hématologiques (baisse du nombre de globules rouges, des globules blancs et des plaquettes), rénaux, auditifs (bourdonnements d'oreille, une hypo-acousie pour les hautes fréquences, une difficulté majeure à s'orienter selon le bruit, une perte de sélectivité de l'oreille) ou encore neurologiques (paresthésie des extrémités avec fourmillements plus ou moins permanents, puis une diminution de sensibilité profonde aboutissant à une ataxie plus ou moins nette) **[15]**. D'autre part, le sel K₂PtCl₄ utilisé dans la préparation du cis-platine est deux fois plus onéreux que d'autres sels métalliques correspondants, par exemple le sel d'iridium IrCl₃.xH₂O.

Miller et al. (Organometallics, 4 : 533-538, 1985) **[20]** décrit des complexes de cobalt de formule générale (η⁵-C₅Me₅)Co(bb')C₆H₄ où b et b' représentent indépendamment l'un de l'autre un atome d'oxygène, de soufre, d'azote ou de sélénium et sont liés au cobalt.

Le Brevet US 5,760,260 **[21]** décrit un procédé amélioré de cyclotrimérisation des alcynes [2+2+2] en solutions aqueuses utilisant des complexes cyclopentadiényles de cobalt pour la synthèse de composés organiques pouvant notamment être utiles dans la prévention ou le traitement du cancer.C'est donc un but de la présente invention de fournir de nouveaux complexes organométalliques stables, peu onéreux, présentant une activité anti-cancéreuse et/ou dont les effets secondaires sont réduits. C'est également un but de la présente invention de fournir les moyens de production de tels complexes.

### Description de l'invention

Les Inventeurs de la présente invention ont mis au point de manière inattendue un procédé original de synthèse, fiable, peu coûteux, reproductible, facile à mettre en oeuvre, permettant l'obtention des premiers complexes organométalliques stables dérivés de sélénoquinone. Ils ont également découvert de manière tout à fait inattendue que ces complexes, qui jusqu'à présent n'avaient pas été isolés ni caractérisés, présentaient des propriétés biologiques (cytotoxicité) au moins équivalentes à celles du cis-platine ; les rendant de ce fait utilisables dans le cadre de la prévention ou du traitement de maladies impliquant une prolifération cellulaire anormale, notamment du cancer.

Par « stable », on entend de préférence des complexes qui sont suffisamment stables pour permettre leur préparation, et dont l'intégrité est maintenue pendant une durée suffisante pour permettre leur détection, et de préférece pendant une durée suffisante pour être utilisables dans le cadre de la prévention ou du traitement de maladies impliquant une prolifération cellulaire anormale, notamment du cancer.

Par « prolifération cellulaire anormale », on entend une prolifération qui est indépendante des mécanismes de régulations normaux, par exemple l'arrêt de prolifération cellulaire dû à la mise en jeu de l'apoptose (mort cellulaire programmée).

La présente invention se rapporte donc en premier lieu à un composé organométallique isolé de formule générale (I) :

(C₅Me₅)M(η⁴-C₆E¹E²R³R⁴R⁵R⁶) (I)

ou un sel pharmaceutiquement acceptable de celui-ci ;
dans laquelle :
- M représente un métal Ru, Co, Rh ou Ir ;
- E¹ représente un de sélénium, ou E¹ représente un atome de soufre ou d'oxygène lorsque R³, R⁴, R⁵ et R⁶ représentent chacun un atome d'hydrogène ;
- E² représente un atome de sélénium ;
- R³ et R⁵ représentent chacun un atome d'hydrogène, et R⁴ et R⁶ représentent chacun un groupe alkyle en C₁₋₈, ou R³, R⁴, R⁵ et R⁶ représentent chacun un atome d'hydrogène.

Par « isolé », on entend généralement un composé qui est (i) séparé d'au moins un composé avec lequel il est associé dans la nature et/ou (ii) produit, préparé ou fabriqué par les soins de l'homme.

Par «η»⁴, on entend l'«hapticité» 4, c'est-à-dire que l'arène est lié au métal par quatre liaisons.

Les groupes alkyles peuvent comprendre de 1 à 8 atomes de carbone, de préférence de 1 à 6 atomes de carbone, et en particulier de 1 à 2 atomes de carbone.

Sauf mention contraire, les groupes alkyles peuvent être linéaires, ramifiés ou cycliques.

Selon la présente invention, E¹ et E² représentent chacun un atome de sélénium, et R³, R⁴, R⁵ et R⁶ sont tels que définis ci-dessus. Par exemple, E¹ et E² représentent chacun un atome de sélénium, R³ et R⁵ représentent chacun un atome d'hydrogène, et R⁴ et R⁶ représentent chacun un groupe méthyle.

Selon la présente invention, E¹ et E² représentent chacun un atome de sélénium, et R³, R⁴, R⁵ et R⁶ représentent chacun un atome d'hydrogène.

Selon la présente invention, E¹ représente un atome de soufre ou d'oxygène, de préférence un atome d'oxygène, E² représente un atome de sélénium, et R³, R⁴, R⁵ et R⁶ représentent chacun un atome d'hydrogène.

Selon la présente invention, M représente Ir.

Selon la présente invention, des complexes organométalliques de la présente invention, E¹ et E² sont en position *ortho-* ou *para-.*

Selon la présente invention, lesdits complexes ont l'une des structures suivantes :

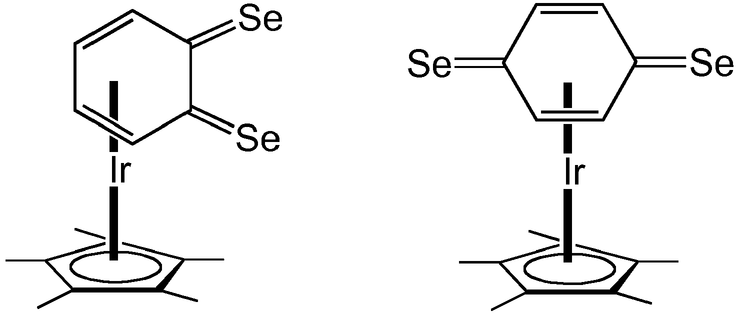

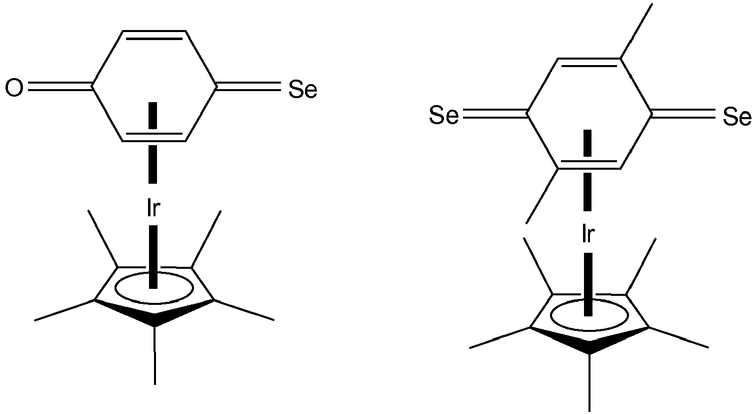

Les Inventeurs ont en outre démontré que les complexes organométalliques de la présente invention possède des propriétés biologiques (cytotoxicité) au moins équivalentes à celles du cis-platine.

Ainsi, selon un mode particulier de mise en oeuvre de la présente invention, les complexes organométalliques de la présente invention peuvent être utilisés comme médicament, notamment pour l'obtention d'un médicament destiné au traitement du cancer.

Les complexes organométalliques de la présente invention peuvent le cas échéant être sous forme solvatée de sel, ou d'autres dérivés physiologiquement acceptables. Les sels et les solvants qui sont acceptables pour une utilisation pharmaceutique sont généralement ceux dans lesquels le contre ion ou le solvant associé est pharmaceutiquement acceptable.

Les sels utilisables peuvent être des acides ou des bases organiques ou minérales. Parmi les sels d'addition acides acceptables, on peut citer ceux formés à partir d'acide chlorhydrique, bromhydrique, sulfurique, citrique, tartrique, phosphorique, lactique, pyruvique, acétique, trifluoroacétique, phénylacétique, triphénylacétique.

On peut également citer parmi les sels basiques acceptables, les sels de métaux alcalins, tels que le sodium ou le potassium, les sels de métaux alcalino-terreux, tels que le calcium et le magnésium, et les sels formés à partir de bases organiques, tels que les amines mono-, di- ou tri-substituées.

La présente invention se rapporte également à une composition pharmaceutique comprenant à titre de principe actif au moins un composé selon la présente invention dans un support pharmaceutiquement acceptable.

En particulier, la composition pharmaceutique peut être anti-cancéreuse.

Dans la composition pharmaceutique, les composés sont employés en quantité efficace. Celle-ci sera déterminée par l'homme du métier, selon différents paramètres, en particulier par rapport à la substance utilisée, l'âge, le poids, et l'état physique du patient, le mode d'administration, et le régime requis. L'Homme du Métier sera à même de déterminer le mode d'administration et le dosage pour chaque patient.

Tout particulièrement, le composé selon l'invention peut être administré à une dose allant de 0,1 à 5 000 mg par jour et par patient.

La composition pharmaceutique peut comprendre une quantité en composé selon l'invention allant de 0,1 mg à 5 g.

La composition pharmaceutique peut être administrée sous toute forme, topique ou systémique, notamment sous forme parentérale ou entérale.

Lorsque la composition ou le médicament est administré(e) par voie entérale, il(elle) peut se présenter sous forme de comprimés, de gélules, de dragées, de sirops, de suspensions, de solutions, de poudres, de granulés, d'émulsions, de microsphères.

Dans le cas d'une administration par voie parentérale, la composition peut se présenter sous forme de solutions ou de suspensions pour perfusion ou pour injection.

La composition peut comprendre en outre au moins un additif, choisi notamment parmi les agents de couleur, de saveur, et les conservateurs. Bien entendu, l'Homme du métier veillera à choisir le ou les additif(s) de manière à ce que les propriétés avantageuses attachées intrinsèquement à l'invention ne soient pas, ou substantiellement pas, altérées par l'addition envisagée.

Selon un mode de réalisation particulier La composition selon l'invention peut comprendre en outre un autre composé destiné à traiter le cancer. Parmi les composés utilisables selon l'invention, on peut citer la doxorubicine, dont la dénomination commerciale est l'adriamycine^{®}, l'épothilone, le paclitaxel, dont la dénomination commerciale est le taxol^{®} et le cis-platine.

Selon encore un autre de ses aspects, l'invention à pour objet l'utilisation d'au moins un complexe organométallique de la présente invention pour la préparation d'une composition pharmaceutique destinée à traiter et/ou à prévenir une maladie impliquant une prolifération cellulaire anormale, notamment un cancer.

Ladite composition peut être destinée à la médecine humaine et/ou vétérinaire, et en particulier elle peut être destinée à traiter ou à prévenir au moins un cancer choisi par exemple parmi le cancer du pancréas, les cancers de l'oro-pharynx, le cancer de l'estomac, le cancer de l'oesophage, le cancer du colon et rectal, le cancer du cerveau, notamment les gliomes, le cancer des ovaires, le cancer du foie, le cancer du rein, le cancer du larynx, le cancer de la thyroïde, le cancer du poumon, le cancer des os, les myélomes multiples, les mésothéliomes et les mélanomes, le cancer de la peau, le cancer du sein, le cancer de la prostate, le cancer de la vessie, le cancer de l'utérus, le cancer des testicules, les lymphomes non-Hodgkinien, la leucémie, la maladie de Hodgkin, et des cancers des tissus mous, ainsi que des localisations secondaires métastatiques des cancers cités précédemment.

La présente invention se rapporte également à un procédé de synthèse des complexes organométalliques de la présente invention qui comprend une attaque nucléophile du composé de formule (II) suivante :

L(C₅Me₅)M(η⁶-C₆X¹X²R³R⁴R⁵R⁶)][Z]₂ (II)

dans laquelle :
M, R³, R⁴, R⁵ et R⁶ sont tels que définis ci-dessus ;
X¹ et X² sont identiques ou différents et représentent chacun un atome d'halogène Cl, Br, ou I ;
Z représente un contre-anion BF₄⁻, PF₆⁻ ou CF₃SO₃⁻ (ou OTf⁻) ;
par au moins un nucléophile Y₂Se où Y est un cation de métal alcalin.

Selon un mode particulier de réalisation du procédé de synthèse de la présente invention, l'attaque nucléophile est réalisée avec du séléniure de sodium (Na₂Se).

D'autres avantages pourront encore apparaître à l'homme du métier à la lecture des exemples ci-dessous, illustrés par les figures annexées, donnés à titre illustratif.

### Brève description des figures

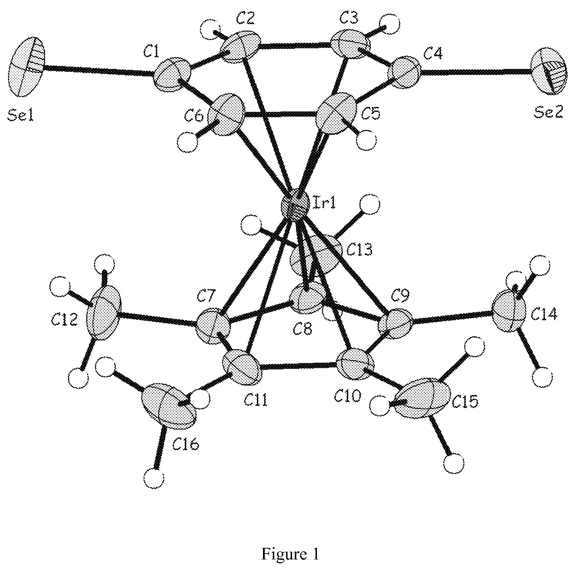

- La figure 1 représente la structure cristallographique du complexe organométallique [(C₅Me₅)Ir(η⁴-*p*-diselenobenzoquinone)] (8)

### EXEMPLES

### Exemple 1 : Synthèse de complexes iridium sélénoquinone 7-8

Il existe trois procédés de synthèse différents pour préparer les complexes quinone (1-4) dithioquinone (5,6) et mono- et disélénoquinone (7, 8, 9, 10). La synthèse des familles 1-6 est bien connue de l'art **[13]**, contrairement à celle des complexes mono- et disélénoquinone qui constitue un objet de la présente invention.

### 1- Synthèse des complexes iridium quinone (1-4)

Des complexes organométalliques *para*-quinone ont été préparés par traitement direct de l'hydroquinone et du composé solvaté [(C₅Me₅)M(solvant)₃]²⁺ où M=Rh ou Ir préparé *in situ,* suivi d'une déprotonation subséquente par une base afin d'obtenir les complexes organométalliques paraquinone *p*-[(C₅Me₅)M(η⁴-quinone)] où M=Rh (2) ou Ir (4) **[8-9]**.

De manière similaire, des complexes organométalliques *ortho*-quinone ont été obtenus par taitement du catéchol avec le composé solvaté [(C₅Me₅)M(solvant)₃]²⁺ où M=Rh ou Ir, en présence de BF₃.2H₂O en tant qu'activateur de l'arène afin d'assurer la liaison du fragment métallique au niveau du cycle arène, suivi d'une déprotonation afin d'obtenir les complexes organométalliques *ortho*-quinone *o*-[(C₅Me₅)M(η⁴⁻quinone)] où M=Rh (1) ou Ir (3) **[11]**.

### 2- Synthèse des complexes iridium dithioquinone (5,6)

Les homologues soufrés de l'hydroquinone et du catéchol, ont été obtenus par un procédé de synthèse différent de ci-dessus. Ainsi, les complexes organométalliques dichloro *ortho-* et *para*-benzene sensibles à l'air ont été préparés et isolés sous forme de composés microcristallins blancs. Un traitement desdits composés avec de l'hydrosulfure de sodium a produit les complexes organométalliques thioquinone sous forme d'isomères *ortho-* (5) et *para-* (6) **[12]**.

### 3- Synthèse des complexes iridium mono- et di-sélénoquinone (7, 8, 9, 10)

Les complexes organométalliques *ortho-* et *para-*sélénoquinone ont été synthétisés selon le schéma réactionnel ci-dessous :

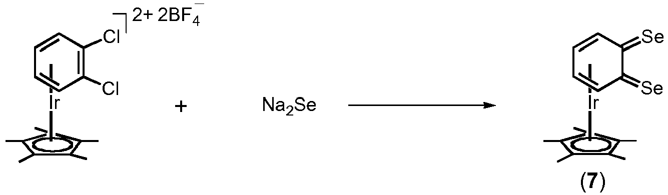

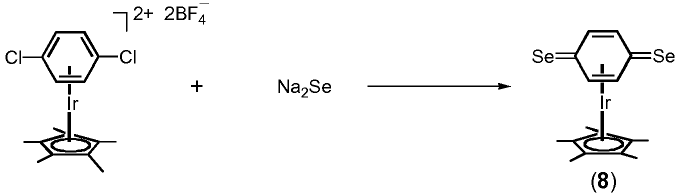

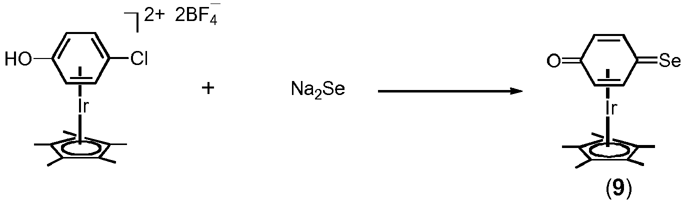

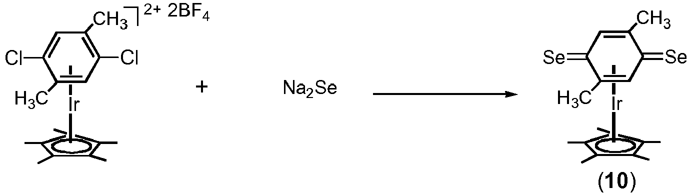

La première étape de ce schéma de synthèse implique la préparation de complexes organométalliques de dichloro- ortho- et para-benzène halogénés sensibles à l'air [(C₅Me₅)Ir(η⁶-C₆H₄Cl₂)][BF₄]₂, de monochloro-para-benzène halogéné sensible à l'air *p*-[(C₅Me₅)Ir(η⁶-C₆H4ClOH)][BF₄]₂, et de dichloro-diméthyl-para-benzène halogéné sensible à l'air *p*-[(C₅Me₅)Ir(η⁶-C₆H₂Cl₂(CH₃)₂)][BF₄]₂.

### 3-1- Synthèse du complexe p-[(C₅Me₅)Ir(η⁴-diselenobenzoquinone)] (8)

A un tube Schlenk contenant du Na₂Se anhydre (650mg, 5,2mmol), conservé sous atmosphère d'argon, a été ajoutée une solution colorée de *p*-[(C₅Me₅)Ir(η⁶-C₆H₄Cl₂)][BF₄]₂ (340mg, 0,52mmol) dans du CH₃CN distillé extemporanément (10ml). Le mélange réactionnel a viré à l'orange rapidement avec la formation d'un précipité (NaCl et NaBF₄). La réaction a été maintenue pendant 20 minutes, puis le solvant a été éliminé sous vide afin de fournir un résidu noir orangé. Par la suite, le composé a été extrait à l'aide de 50ml de CH₂Cl₂ distillé, et filtré sous atmosphère d'argon à travers une fritte équipée de coton, célite, coton pour donner une couleur orange brilllante. Le solvant a été éliminé sous vide pour donner une poudre microcristalline orange identifiée comme le *p*-[(C₅Me₅)Ir(η⁴-C₆H₄Se₂)][BF₄]₂ (241 mg, 0,49mmol). Rendement 95%.

Le composé (8) est stable et peut être conservé pendant une longue période sous atmosphère d'argon. Le composé (8) est soluble dans CH₂Cl₂, MeOH, l'acétone et dans la plupart des solvants organiques polaires.
IR (ATR), v cm⁻¹. 2990, 1467, 1421, 1380, 1272, 1053, 1024, 731, 704, 633,431,353.
¹H NMR (400MHz, CD₂Cl₂), δ (ppm) : 1,89 (15H, s, η-C₅Me₅) ; 6,26 (4H, s, CH-p-diselenobenzoquinone).
¹³C{¹H} NMR (100MHz, CD₂Cl₂), δ (ppm) : 6,86 (CH3, s, η-C₅Me₅) ; 96,83, (CH, s, p-disélénoquinone) 96,97 (C=C, s, η-C₅Me₅) ; 132,96 (C-Se, s, disélénoquinone).
⁷⁷Se{¹H} NMR (400MHz, CD₂Cl₂), δ (ppm) : 296 (2Se, s, C-Se)

### 3-2- Synthèse du complexe o-[(C₅Me₅)Ir(η⁴-diselenobenzoquinone)] (7)

Le composé (7) a été préparé de manière similaire à celle du composé (8). La molécule est un peu moins stable que l'isomère *para-.* Actuellement des études sont en cours pour optimiser la méthode de synthèse.
IR (ATR), v cm⁻¹. 3373, 2912, 1585, 1467, 1378, 1258, 1019, 885, 800, 728,696,636,608,550,518,464,386,308.

### 3-3- Synthèse du complexe p-[(C₅Me₅)Ir(η⁴-monoselenobenzoquinone)] (9)

Le composé (9) a été préparé de manière similaire à celle du composé (8) mais à partir du complexe organométallique mono-chloré *p*-[(C₅Me₅)Ir(η⁶-C₆H₄ClOH)][BF₄]₂, et a été obtenu avec un rendement de 90%.
¹H NMR (400MHz, CD₂Cl₂), δ (ppm) : 1, 92 (15H, s, η-C₅Me₅) ; 5,32 (2H, d, 6Hz, CH-p-diselenobenzoquinone); 6.29 (2H, d, 6Hz, CH-p-diselenobenzoquinone).
IR (ATR), v cm⁻¹. 2964, 2917, 1630, 1605, 1468, 1385, 1259, 1024, 802, 691,636,606,557,518,452,429.

### 3-4- Synthèse du complexe p-[(C₅Me₅)Ir(η⁴-2,5-diméthyl-diselenobenzoquinone)] (10)

Le composé (10) a été préparé de manière similaire à celle du complexe 8 mais à partir de *p*-[(C₅Me₅)Ir(η⁶-C₆H₂Cl₂(CH₃)₂)][BF₄]₂, et a été obtenu avec un rendement de 90%.
¹H NMR (400MHz, MeOH-d₄), δ (ppm) : 1,79 (15H, s, η-C₅Me₅) ; 2.51 (6H, s, CH₃-); 6,78 (2H, s, CH-p-diselenobenzoquinone).
IR (ATR), v cm⁻¹. 3616, 3568, 1637, 1467, 1406, 1381, 1259, 1043, 893, 742, 634, 606, 522, 461.

### Exemple 2 : Propriétés biologiques des complexes organométalliques quinone, thioquinone et sélénoquinone (1-10)

Les propriétés biologiques (cytotoxicité) des complexes synthétisés ci-dessus ont été testées sur les cellules de cancer ovarien A2780 et A2780cisR (résistantes au cis-platine) selon une procédure conventionnelle décrite ci-dessous, et les valeurs IC50 obtenues ont été comparées à celles obtenues dans les mêmes conditions avec le complexe cis-platine Pt(NH₃)₂Cl₂.

Les lignées cellulaires A2780 et A2780cisR de cancer ovarien ont été obtenues de The European Collection of Cell Cultures (ECACC) (Salisbury, UK). Les cellules ont été cultivées en milieu RPMI contenant du glucose, 5% de sérum de veau foetal (SVF) et des antibiotiques, à 37°C et 5% CO2.

La cytotoxicité a été déterminée en utilisant le test au MTT (3-(4,5-dimethyl-2-thiazolyl)-2,5-diphenyl-2H-tetrazolium bromide) (MOSMANN, 1983).

Les cellules ont été réparties dans des plaques 96 puits, en monocouches, avec 100 µl de solution (environ 20 000 cellules) par puits, et ont été pré-incubées pendant 24 heures dans un milieu supplémenté avec 10% de SVF.

Les composés ont été préparés en solution DMSO puis ont été dissous dans le milieu de culture et dilués successivement à la concentration appropriée, afin d'obtenir une concentration finale en DMSO de 0,5%. 100 µl de solution de ces composés ont été ajoutés à chaque puits, et les plaques ont été incubées pendant 72 heures. Par la suite, du MTT (5 mg/ml) a été ajouté aux cellules, et les plaques ont été incubées pendant 2 heures. Le milieu de culture a été éliminé par aspiration, et les cristaux violet de formazan formés par l'activité déshydrogénase mitochondriale des cellules vivantes ont été dissous dans le DMSO.

La densité optique, directement proportionnelle au nombre de cellules vivantes, a été quantifiée à 540 nm en utilisant un lecteur de plaques multipuits, et la fraction de cellules vivantes a été quantifiée à partir de l'absorbance de cellules contrôle non traitées. L'évaluation a été réalisée à partir de deux expérimentations indépendantes ; chacune comprenant 3 microcultures par niveau de concentration.

Les valeurs IC50 obtenues sont présentées dans le tableau 1 ci-dessous.

**Tableau 1**

| **Nom du complexe** | **IC50 (µmol)** |
|---|---|
| o-[(C₅Me₅)Rh(η⁴-benzoquinone)] (1) | >400 |
| p-[(C₅Me₅)Rh(η⁴-benzoquinone)] (2) | >400 |
| o-[(C₅Me₅)Ir(η⁴-benzoquinone)] (3) | >400 |
| p-[(C₅Me₅)Ir(η⁴-benzoquinone)] (4) | 93 |
| o-[(C₅Me₅)Ir(η⁴-dithiobenzoquinone)] (5) | 49 |
| p-[(C₅Me₅)Ir(η⁴-dithiobenzoquinone)] (6) | 154 |
| o-[(C₅Me₅)Ir(η⁴-diselenobenzoquinone)] (7) | / |
| p-[(C₅Me₅)Ir(η⁴-diselenobenzoquinone)] (8) | 5 |
| p-[(C₅Me₅)Ir(η⁴-mono-selenobenzoquinone)] (9) | **12.3** |
| p-[(C₅Me₅)Ir(η⁴-2,5-dimethyl-diselenobenzoquinone)] (10) | **6.2** |
| Cis-(Pt(NH₃)₂Cl₂] | 3 |

Les résultats montrent que les complexes dithiobenzoquinone, et en particulier les complexes organométalliques mono-sélénoquinone et disélénoquinone (8-10), possèdent des propriétés biologiques (cytotoxicité) importantes, équivalentes à celles du composé cis-platine vis-à-vis de la lignée cellulaire A2780 de cancer ovarien.

En outre, de façon tout à fait remarquable, les deux complexes 9 et 10 possèdent des propriétés cytotoxiques supérieures au cis-platine vis-à-vis de la lignée cellulaire A2780cisR (résistante au cis-platine) de cancer ovarien, avec des valeurs IC50 de 8,4 µmol pour le composé 9 et IC50 de 7,3 µmol pour le composé 10, alors que la valeur IC50 du complexe cis-[Pt(NH₃)₂Cl₂] pour la lignée cellulaire A2780cisR est de 25 µmol.

Les complexes organométalliques de l'invention sont donc utilisables dans le cadre de la prévention et/ou du traitement de maladies impliquant une prolifération cellulaire anormale, notamment du cancer. Leur utilisation sera tout particulièrement bénéfique dans le cadre de maladies pour lesquelles un traitement à base de cis-platine s'est révélé peu efficace voire inefficace.

### Exemple 3 : Cristallographie du complexe diselenobenzoquinone p-[(C₅Me₅)Ir(η⁴-diselenobenzoquinone)] (8)

Des cristaux de la molécule biologiquement active [(C₅Me₅)Ir(η⁴-*p*-diselenobenzoquinone)] (**8**), ont été obtenus par diffusion lente d'éther éthylique dans une solution de méthanol de ce complexe. La diffraction aux rayons X a permis la résolution de la structure.

Pour ce faire, la structure a été résolue par des méthodes directes utilisant le programme SIR92 **[18]**, et affinée de manière anisotropique par la méthode des moindres carrés à matrice pleine en utilisant le progiciel SHELXL-97 **[19]**.

La structure résolue par diffraction de rayons X (figure 1) montre que ce complexe cristallise dans une maille monoclinique avec le groupe d'espace P2₁/c et Z = 4.

L'analyse des distances et angles [tableau 2 : sélection de distances*(Å) et angles (°)*] a permis de confirmer la formation de la p-diselenobenzoquinone. Ces données ont indiqué que la p-diselenobenzoquinone est coordonnée à l'iridium par les 4 atomes de carbones dièniques C(2), C(3), C(5) et C(6). En effet, les distances Ir(1)-C(1) et Ir(1)-C(4) sont plus longues que les distances Ir(1)-C(2), Ir(1)-C(3) Ir(1)-C(5) et Ir(1)-C(6). Par conséquent, la *p*-diselenobenzoquinone a adopté une conformation légèrement coudée en forme de bateau. L'angle dièdre entre les plans [C(2)C(3)C(5)C(6)] et [C(2)C(1)C(6)] est égal à 7.04°. Celui entre les plans [C(2)C(3)C(5)C(6)] et [C(3)C(4)C(5)] est égal à 5.94°. En outre, les distances C(1)-Se(1) et C(4)-Se(2) sont égales à 1.876 Å et 1.865 Å respectivement. Ces distances sont en accord avec un caractère plutôt double que simple de la liaison entre C et Se en comparaison avec la distance C-Se trouvée pour le Se-Ar (1.925 Å) **[16]** et la distance C=Se (1.88 Å) **[17]** rencontrée dans le complexe du cobalt [CoCl₂(C₅H₈N₂Se)₂].

**Tableau 2**

| | Å (°) | | Å (°) |
|---|---|---|---|
| **Ir(1)-C(1)** | 2.370(8) | C(1)-Se(1) | 1.876(10) |
| Ir(1)-C(2) | 2.229(8) | C(4)-Se(2) | 1.865(9) |
| Ir(1)-C(3) | 2.236(6) | C(2)-C(1)-C(6) | 113.79(7) |
| **Ir(1)-C(4)** | 2.349(5) | C(3)-C(4)-C(5) | 113.74(7) |
| Ir(1)-C(5) | 2.226(7) | | |
| Ir(1)-C(6) | 2.239(7) | | |

Par ailleurs, l'examen de la structure au niveau du cristal a montré que ce complexe s'empile par des interactions π-π entre les molécules du [(C₅Me₅)Ir(η⁴-C₆H₄Se₂)]. D'une part, le cycle aromatique du ligand Cp* interagit avec le système diènique de la p-diselenobenzoquinone d'une molécule voisine (d = 3.563 A). Ces interactions permettent la formation d'une chaîne supramoléculaire dont la cohésion est assurée par les empilements π-π.

### Listes des références

[1] (a) Larsen, P. L.; Clarke, C. F. Science 2002, 295, 120. (b) Do, T. Q.; Hsu, A. Y.; Jonassen, T.; Lee, P. T.; Clarke, C. F. J. Biol. Chem. 2001, 276, 18161. (c) Steinberg-Yfrach, G.; Liddell, P. A.; Moore, A. L.; Gust, D.; Moore, T. A. Nature 1997, 385, 239. (d) Cross, J. V.; Deak, J. C.; Rich, E. A.; Qian, Y.; Lewis, M.; Parrott, L. A.; Mochida, K.; Gustfason, D.; Vande Pol, S.; Templeton, D. J. J. Biol. Chem. 1999, 274, 31150.
[2] Lamson, D. W.; Plaza, S. M. Altern. Med. Rev. 2003, 8, 303.
[3] Voet, D.; Voet, J. B.; Pratt, C. W. Fundamentals of Biochemistry, Wiley: New York, 1999.
[4] (a) Parker, V. Chem. Commun. 1969, 716. (b) Eggins, B.; Chambers, J. Q. Chem. Commun. 1969, 232.
[5] (a) Liebeskind, L. S.; Jewell, C. F. Jr. J. Organomet. Chem. 1985, 285, 305. (b) Jewell, C. F. Jr.; Liebeskind, L. S.; Williamson, M. J. Am. Chem. Soc. 1985, 107, 6715*.* (c) Cho, S. H.; Wirtz, K. R.; Liebeskind, L. S. Organometallics 1990, 9, 3067.
[6] (a) Oh, M.; Carpenter, G. B.; Sweigart, D. A. Organometallics, 2002, 21, 1290. (b) Ura, Y.; Sato, Y.; Shiotsuki, M.; Suzuki, T.; Wada, K.; Kondo, T.; Mitsudo, T. Organometallics, 2003, 22, 77.
[7] (a) Wright, M. E.; J. Organomet. Chem. 1989, 376, 353. (b) Schumann, H.; Arif, A. M.; Richmond, T. G. Polyhedron, 1990, 9, 1677.
[8] (a) Le Bras, J.; Amouri, H.; Vaissermann, J. Organometallics 1998, 17, 1116. (b) Le Bras, J.; Amouri, H.; Vaissermann, J. J. Organomet. Chem. 1998, 553, 483.
[9] Moussa, J.; Guyard-Duhayon, C.; Herson, P.; Amouri, H.; Rager, M. N.; Jutand, A. Organometallics, 2004, 23, 6231.
[10] (a) Bock, H.; Mohmand, S.; Hirabayashi, T.; Maier, G.; Reisenauer, H. P. Chem. Ber. 1983, 116, 273. (b) Breitenstein, M.; Schulz, R.; Schweig, A. J. Org. Chem. 1982, 47, 1979.
[11] Moussa, J.; Rager, M. N.; Chamoreau, L.-M.: Ricard, L.; Amouri, H. Organometallics, 2009, 28, 397.
[12] (a) Moussa, J.; Lev, D. A.; Boubekeur, K.; Rager, M. N.; Amouri, H. Angew. Chem. Int. Ed. 2006, 45, 3854. (b) Moussa, J.; Rager, M. N.; Boubekeur, K.; Amouri H. Eur. J. Inorg. Chem. 2007, 2648.
[13] Moussa, J.; Amouri, H. Angew. Chem. Int. Ed. 2008, 47, 1372.
[14] Bellon SF.; Coleman JH.; Lippard SJ. Biochem. 1991, 30, 8026
[15] Reedijk J.; Eur. J. Inorg. Chem. 2009, 2009, 1283
[16] J. M. White, J. B. Lambert, M. Spiniello, S. A. Jones, R. W. Gable Chem. Eur. J. 2002, 8, 2799.
[17] D. J. Williams, T. A. Jones, E. D. Rice, K. J. Davis, J. A. Ritchie, W. T. Pennington, G. L. Schimek Acta Cryst. 1997, C53, 837.
[18] A. Altomare, G. Cascarano, C. Giacovazzo and A. Guagliardi, J. Appl. Crystallogr. 1993, 26, 343
[19] G. M. Sheldrick, Acta Cryst. A, 2008, 64, 112
[20] Miller, J. ; Landon, S.J. ; Brill, T.B. Organometallics. 1985, 4, 533
[21] Brevet US 5,760,260

## Revendications

1. Complexe organométallique isolé de formule générale (I) :
(C₅Me₅) M(η⁴-C₆E¹E²R³R⁴R⁵R⁶) (I)
ou un sel pharmaceutiquement acceptable de celui-ci ;
dans laquelle :
- M représente un métal Ru, Co, Rh ou Ir ;
- E¹ représente un atome de sélénium, ou E1 représente un atome de soufre ou d'oxygène lorsque R³, R⁴, R⁵ et R⁶ représentent chacun un atome d'hydrogène ;
- E² représente un atome de sélénium ;
- R³ et R⁵ représentent chacun un atome d'hydrogène et R⁴ et R⁶ représentent chacun un groupe alkyle en C₁₋₈, ou R³, R⁴, R⁵ et R⁶ représentent chacun un atome d'hydrogène.

2. Complexe selon la revendication 1, où le groupe alkyle en C₁₋₈ est un groupe méthyle.

3. Complexe selon l'une quelconque des revendications 1 ou 2, où M représente Ir.

4. Complexe selon l'une quelconque des revendications 1 à 3, où E¹ et E² sont en position *ortho-* ou *para-.*

5. Complexe selon la revendication 1, ayant l'une des structures suivantes :

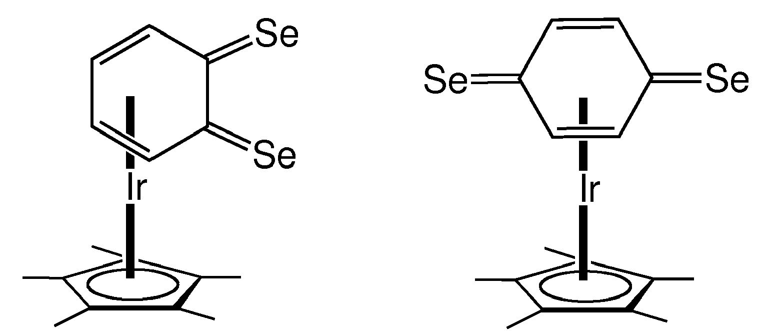

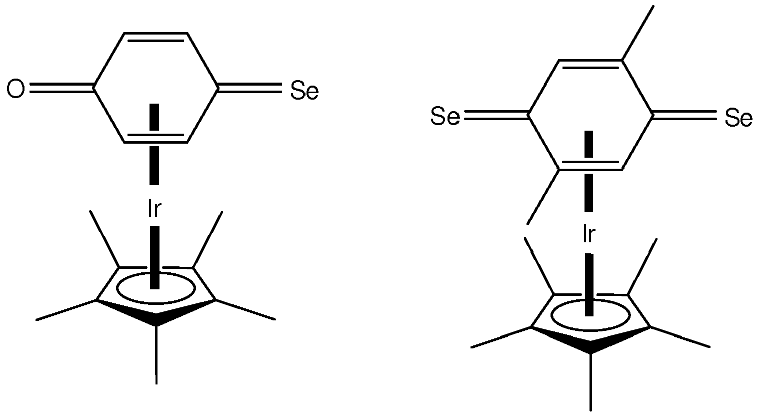

6. Composition pharmaceutique comprenant à titre de principe actif au moins un complexe selon l'une quelconque des revendication 1 à 5 dans un support pharmaceutiquement acceptable.

7. Complexe selon l'une quelconque des revendications 1 à 5 pour une utilisation comme médicament.

8. Complexe selon la revendication 7, où ledit médicament est destiné à la prévention ou au traitement du cancer.

9. Complexe selon l'une quelconque des revendications 1 à 5 pour l'obtention d'une composition pharmaceutique destinée à la prévention ou au traitement du cancer.

10. Procédé de synthèse d'un complexe selon l'une quelconque des revendications 1 à 5, **caractérisé en ce qu'**il comprend une attaque nucléophile du complexe de formule (II) suivante :
[(C₅Me₅)M(η⁶-C₆X¹X²R³R⁴R⁵R⁶)][Z]₂ (II)
dans laquelle :
- M, R³, R⁴, R⁵ et R⁶ sont tels que définis dans les revendications 1 ou 2 ;
- X¹ et X² sont identiques ou différents et représentent chacun un atome d'halogène CI, Br, ou I ;
- Z représente un contre-anion anion BF₄⁻, PF₆⁻ ou CF₃SO₃⁻;
par au moins un nucléophile Y₂Se où Y est un cation de métal alcalin.

11. Procédé de synthèse selon la revendication 10, où l'attaque nucléophile est réalisée avec du séléniure de sodium (Na₂Se).

## Patentansprüche

1. Isolierter metallorganischer Komplex mit der folgenden allgemeinen Formel (I):
(C₅Me₅) M(η⁴-C₆E¹E²R³R⁴R⁵R⁶) (I)
oder ein pharmazeutisch annehmbares Salz davon;
wobei:
- M ein Metall Ru, Co, Rh oder Ir darstellt;
- E¹ ein Selenatom darstellt, oder E1 ein Schwefel- oder Sauerstoffatom darstellt, wenn R³, R⁴, R⁵ und R⁶ jeweils ein Wasserstoffatom darstellen;
- E² ein Selenatom darstellt;
- R³ und R⁵ jeweils ein Wasserstoffatom darstellen und R⁴ und R⁶ jeweils eine C₁₋₈-Alkylgruppe darstellen, oder R³, R⁴, R⁵ und R⁶ jeweils ein Wasserstoffatom darstellen.

2. Komplex nach Anspruch 1, wobei die C₁₋₈-Alkylgrupppe eine Methylgruppe ist.

3. Komplex nach einem der Ansprüche 1 oder 2, wobei M Ir darstellt.

4. Komplex nach einem der Ansprüche 1 bis 3, wobei E¹ und E² in der *ortho-* oder *para*-Position sind.

5. Komplex nach Anspruch 1, umfassend eine der folgenden Strukturen:

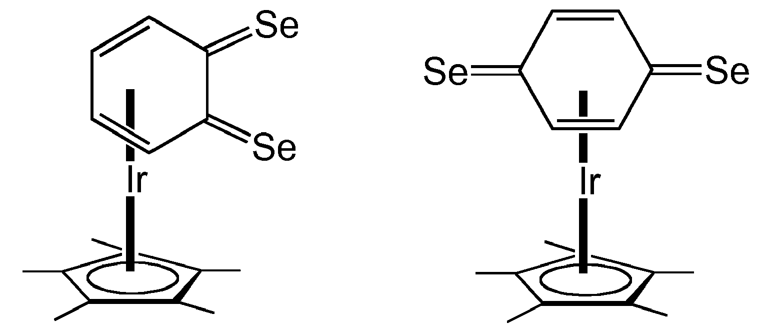

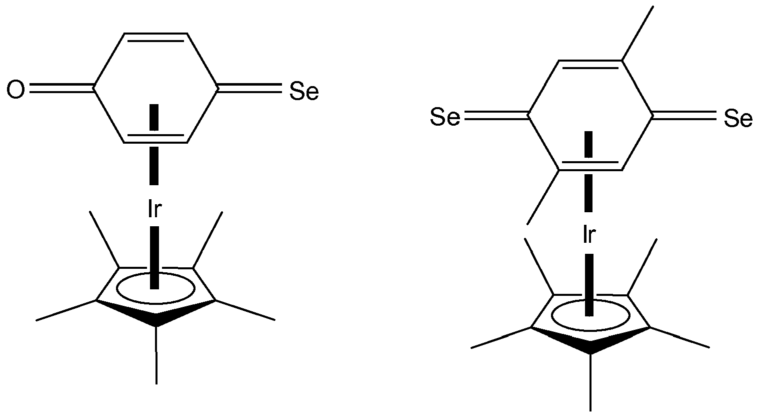

6. Pharmazeutische Zusammensetzung, umfassend als Wirkstoff mindestens einen Komplex nach einem der Ansprüche 1 bis 5, in einem pharmazeutisch akzeptablen Träger.

7. Komplex nach einem der Ansprüche 1 bis 5 für eine Verwendung als Arzneimittel.

8. Komplex nach Anspruch 7, wobei das Arzneimittel ausgelegt ist, um Krebs vorzubeugen oder zu behandeln.

9. Komplex nach einem der Ansprüche 1 bis 5 zum Erhalt einer pharmazeutischen Zusammensetzung, die ausgelegt ist, um Krebs vorzubeugen oder zu behandeln.

10. Verfahren zur Synthese eines Komplexes nach einem der Ansprüche 1 bis 5, **dadurch gekennzeichnet, dass** er einen nukleophilen Angriff des Komplexes mit der folgenden Formel (II) umfasst:
[(C₅Me₅)M(η⁶-C₆X¹X²R³R⁴R⁵R⁶)][Z]₂ (II)
wobei:
- M, R³, R⁴, R⁵ und R⁶ wie in Anspruch 1 oder 2 definiert sind;
- X¹ und X² identisch oder verschieden sind und jeweils ein Halogenatom Cl, Br oder I darstellen;
- Z ein Gegenanion BF₄⁻, PF₆⁻ oder CF₃SO₃⁻ darstellt;
durch mindestens ein Nukleophil Y₂Se, wobei Y ein alkalines Metallkation ist.

11. Verfahren zur Synthese nach Anspruch 10, wobei der nukleophile Angriff mit Natriumselenid (Na₂Se) durchgeführt wird.

## Claims

1. An isolated organometallic complex of general formula (I):
(C₅Me₅)M(η⁴-C₆E¹E²R³R⁴R⁵R⁶) (I)
or a pharmaceutically acceptable salt of the latter;
in which:
- M represents a metal Ru, Co, Rh or Ir;
- E¹ represents a selenium atom, or E¹ represents an oxygen or sulfur atom when R³, R⁴, R⁵ and R⁶ each represent a hydrogen atom;
- E² represents a selenium atom;
- R³ and R⁵ each a hydrogen atom and R⁴ and R⁶ each represent a C₁₋₈ alkyl group or R³, R⁴, R⁵ and R⁶ each represent a hydrogen atom.

2. Complex as claimed in claim 1, where the C₁₋₈ alkyl group is a methyl group.

3. Complex as claimed in either one of claims 1 or 2, where M represents Ir.

4. Complex as claimed in any one of claims 1 to 3, where E¹ and E² are in the *ortho-* or *para*-position.

5. Complex as claimed in claim 1, having one of the following structures:

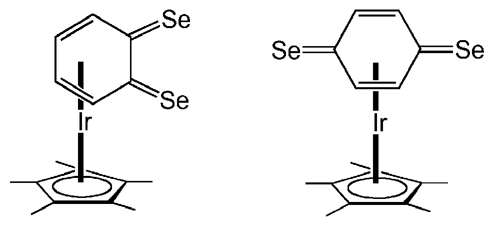

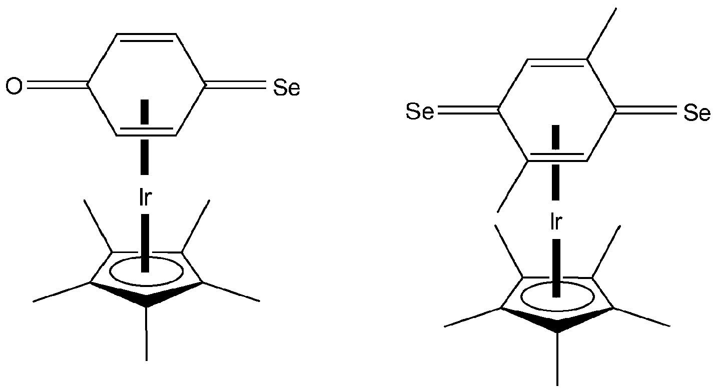

6. Pharmaceutical composition comprising, as active principle, at least one complex as claimed in any one of claims 1 to 5 in a pharmaceutically acceptable vehicle.

7. Complex as claimed in any one of claims 1 to 5 for use as medicament.

8. Complex as claimed in claim 7, where said medicament is intended for the prevention or treatment of cancer.

9. Complex as claimed in any one of claims 1 to 5 for the production of a pharmaceutical composition intended for the prevention or treatment of cancer.

10. Method for the synthesis of a complex as claimed in any one of claims 1 to 5, **characterized in that** it comprises a nucleophilic attack on the complex of following formula (II):
[(C₅Me₅)M(η⁶-C₆X¹X²R³R⁴R⁵R⁶)][Z]₂ (II)
in which:
- M, R³, R⁴, R⁵ and R⁶ are as defined in either one of claims 1 or 2;
- X¹ and X² are identical or different and each represent a halogen atom Cl, Br or I;
- Z represents a counteranion BF₄⁻, PF₆⁻ or CF₃SO₃⁻, by at least one nucleophile Y₂Se, where Y is an alkali metal cation.

11. Method for the synthesis as claimed in claim 10, where the nucleophilic attack is carried out with sodium selenide (Na₂Se).
